# EUROPÄISCHE PATENTANMELDUNG

(11) **EP 1 224 946 A1**
(43) Veröffentlichungstag der Anmeldung: **24.07.2002**
(21) Anmeldenummer: 02000827.2
(22) Anmeldetag: 15.01.2002
(51) Int. Cl.: A61L 2/12, A23L 3/01, F26B 3/347, B27K 7/00, F26B 21/00

(54) **Vorrichtung zur Behandlung eines Schüttguts, insbesondere von Korkgranulat**

(30) Priorität: 18.01.2001 DE 10101980
(71) Anmelder: Linn High Term GmbH, 92275 Eschenfelden (DE); Rudolf Ohlinger GmbH & Co. KG, 67136 Fussgönheim (DE)
(72) Erfinder: Linn, Horst, 92275 Eschenfelden (DE); Ohlinger, Rudolf, 67069 Ludwigshafen (DE)
(74) Vertreter: LOUIS, PÖHLAU, LOHRENTZ & SEGETH

(57) **Zusammenfassung**

Es wird eine Vorrichtung (10) zur Behandlung eines Schüttguts, insbesondere zur Reduktion chemischer Kontaminanten, zur Sterilisation und zur Trocknung von Korkgranulat durch Bestrahlung mit einer Mikrowellenstrahlung beschrieben. Die Vorrichtung (10) weist ein liegendes zylindrisches Ofengehäuse (14) auf, in dessen Innerem (24) ein Rührwerk (26) mit von einer hohlen Rührwerkachse (28) wegstehenden hohlen Rührorganen (30) vorgesehen ist. Das Ofengehäuse (14) weist einen Granulateinlaß (40) und eine Granulatausgabeeinrichtung (42) auf und am Gehäusemantel (16) sind voneinander beabstandet eine Anzahl Mikrowellen-Strahlenquellen (22) angeordnet. Die hohle Rührwerkachse (28) weist einen Einlaßanschluß für ein gasförmiges Fluid und Auslaßlöcher für das gasförmige Fluid auf. Die hohlen Rührorgane (32) sind mit der hohlen Rührwerkachse (28) strömungstechnisch verbunden und können ebenfalls mit Auslaßlöchern ausgebildet sein. Das Ofengehäuse (14) weist außerdem einen Absauganschluß für das gasförmige Fluid auf. Der Absauganschluß kann auch an der hohlen Rührwerkachse (26) vorgesehen sein.

## Beschreibung

Die Erfindung betrifft eine Vorrichtung zum Behandeln eines Schüttguts, insbesondere zur Reduktion chemischer Kontaminanten, zur Sterilisation und zur Trocknung von Korkgranulat durch Bestrahlung mit einer Mikrowellenstrahlung, mit einem liegenden zylindrischen Ofengehäuse, in dessen Innerem ein Rührwerk mit von einer Rührwerkachse wegstehenden Rührorganen vorgesehen ist, wobei das Ofengehäuse einen Granulateinlaß und eine Granulatausgabeeinrichtung aufweist, und wobei am Gehäusemantel voneinander beabstandet eine Anzahl Mikrowellen-Strahlenquellen angeordnet sind.

Bei dem Schüttgut kann es sich beispielsweise auch um ein Nahrungsmittelgranulat, um ein Isolierstoffgranulat, wie beispielsweise Perlite, oder um ein Kunststoffgranulat handeln.

Naturkorken aus der Baumrinde der Korkeiche werden bereits seit dem Altertum zum Verschluß von Flaschen, Rohren, usw. angewandt. Für Flaschenkorken hochwertiger Produkte, wie Wein und Sekt, werden Korken unterschiedlichen Aufbaus verwendet, die entweder aus der Korkrinde der Korkeiche ausgestanzt werden, oder die aus zermahlenem Korkmaterial unter Verwendung von Bindemitteln zu Agglomeratkorken geformt werden. Noch eine andere Möglichkeit besteht darin, Naturkorkscheiben und Agglomeratkörper zu kombinieren. Agglomeratkorken werden üblicherweise aus den vermahlenen, nicht genutzten Resten der Naturkorkproduktion hergestellt. Diese nicht genutzten Reste der Naturkorkproduktion stellen einen Anteil von ca. 60 % einer Korkplatte dar. Das Korkgranulat wird- je nach der zu produzierenden Qualität der Agglomeratkorken - mehr oder weniger stark gereinigt, mit Bindemitteln wie beispielsweise einem PU-Kleber vermischt. Das Gemisch aus Korkgranulat und Bindemittel wird anschließend geformt. Bei dieser Formung kann es sich um ein Extrusionsverfahren oder um ein Einzelstückverfahren handeln.

Im Verlaufe der unterschiedlichen Produktionsprozesse kann es aus verschiedenen Gründen zu einer starken Zunahme von Mikroorganismen auf und im Korkmaterial kommen. Hierbei kann es sich um eine Neukontamination von außen oder um eine Zunahme der Mikroorganismen durch eine Verbesserung ihrer Lebensbedingungen aufgrund einer Temperaturerhöhung und einer Erhöhung der Feuchtigkeit im Inneren der Korken handeln. Parallel dazu kann auf sehr unterschiedlichen Wegen ein Eintrag chemischer Kontaminanten in das Korkmaterial erfolgen. Das Zusammenwirken mikrobieller Stoffwechselaktivität mit der Präsenz chemischer Kontaminanten kann dann zur Bildung intensiv riechender Stoffwechselprodukte führen, die den Verursacher des typischen Korkgeschmacks im Wein bilden. Daneben können chemische Kontaminanten direkt, das heißt ohne eine zwischengeschaltete Metapolysierung, zu Kork-Fehltönen führen.

Ein Behandlungsverfahren, insbesondere ein Dekontaminations- und Sterilisationsverfahren für Kork bzw. Korkmaterial, wobei der Kork bzw. das Korkmaterial einer Mikrowellenbestrahlung mit einer Leistung von 20 - 1000 W/kg Korken während einer Zeit von 5 - 30 Minuten ausgesetzt wird, ist aus der DE 198 02 297 C1 bekannt. Dabei wird das Korkmaterial vorzugsweise in einem kontinuierlich arbeitenden Mikrowellen-Banddurchlaufofen bestrahlt. Ein solcher Banddurchlaufofen eignet sich infolge seiner hohen Anschaffungskosten jedoch nur für große Quantitäten von Korkgranulat.

Eine Vorrichtung der eingangs genannten Art ist aus der DE 41 41 976 C2 bekannt. Diese bekannte Vorrichtung weist ein liegendes zylindrisches Ofengehäuse auf, in dem ein Rührwerk mit von einer Rührwerkachse wegstehenden Rührorganen vorgesehen ist. Das Ofengehäuse weist einen Granulat-Einlaß und eine Granulat-Ausgabeeinrichtung auf. Am Gehäusemantel des Ofengehäuses sind voneinander beabstandet eine Anzahl Mikrowellen-Strahlenquellen angeordnet. Diese bekannte Vorrichtung ist insbesondere zur Behandlung von Gewürzen vorgesehen. Um eine Befeuchtung der zu behandelnden Gewürze zu ermöglichen, ist dort das Ofengehäuse mit Dampfanschlüssen versehen. Diese befinden sich insbesondere an der Unterseite des Ofengehäuses.

Der Erfindung liegt die Aufgabe zugrunde, eine Vorrichtung der eingangs genannten Art zur Behandlung eines Schüttguts, insbesondere zur Reduktion chemischer Kontaminanten, zur Sterilisation und zur Trocknung von Korkgranulat zu schaffen, mit der eine verbesserte Dekontamination, Geruchsneutralisation, Sterilisation und Trocknung von Korkgranulat möglich ist.

Diese Aufgabe wird erfindungsgemäß dadurch gelöst, daß die Rührwerkachse als hohle Achse ausgebildet ist, die einen Einlaßanschluß für ein gasförmiges Fluid aufweist, und daß die hohle Rührwerkachse mit Auslaßlöchem für das gasförmige Fluid ausgebildet ist.

Mit Hilfe des gasförmigen Fluids, bei dem es sich um ein geeignetes Gas oder um Luft handelt, das durch die hohle Achse des Rührwerks durchgeleitet und durch die Auslaßlöcher aus der Rührwerksachse ausströmt, wird die Verwirbelung des Korkgranulates und somit die Reduktion chemischer Kontaminanten, die Geruchsneutralisation, die Sterilisation und die Trocknung des zu behandelnden Korkgranulates verbessert.

Die erfindungsgemäße Vorrichtung ist insbesondere zum Batchbetrieb, d.h. zum chargeweisen Betrieb geeignet. Ein solcher Batchbetrieb kommt bei relativ kleinen Schüttgut- insbesondere Korkgranulat-Quantitäten zur Anwendung.
Selbstverständlich kann die erfindungsgemäße Vorrichtung auch kontinuierlich betrieben werden.

Bei der erfindungsgemäßen Vorrichtung sind die Mikrowellen-Strahlenquellen am Umfang des Gehäusemantels vorzugsweise äquidistant vorgesehen. Dabei können die Mikrowellen-Strahlenquellen direkt und unmittelbar am Umfang des Gehäusemantels angeordnet sein. Auf diese Weise ergibt sich ein konstruktiv einfacher Aufbau der erfindungsgemäßen Vorrichtung.

Eine optimale Wirkung der Mikrowellenstrahlung der Mikrowellen-Strahlenquellen wird erreicht, wenn bei der erfindungsgemäßen Vorrichtung die Mikrowellen-Strahlenquellen entlang einer Schraubenlinie angeordnet sind.

Eine optimale chemische Dekontamination, Geruchsneutralisation, Sterilisation und Trocknung von Korkgranulat ist erzielbar, wenn bei der erfindungsgemäßen Vorrichtung die von der hohlen Rührwerkachse wegstehenden Rührorgane als hohle Rührorgane ausgebildet, mit der Rührwerkachse fluidisch verbunden und mit Auslaßlöchem für das gasförmige Fluid versehen sind. Die von der Rührwerkachse wegstehenden Rührorgane können als Stäbe, Paddel, Schaufeln, Rahmen oder dergleichen gestaltet sein.

Bei der erfindungsgemäßen Vorrichtung kann die hohle Rührwerkachse und/oder das Ofengehäuse mit einem Absauganschluß für das gasförmige Fluid versehen sein.

Der Granulateinlaß ist bei der erfindungsgemäßen Vorrichtung zweckmäßigerweise am Oberrand der einen Stirnseite des liegenden Ofengehäuses und die Granulatausgabeeinrichtung ist zweckmäßigerweise am Gehäusemantel unterseitig in der Nachbarschaft der zweiten Stirnseite des liegenden Ofengehäuses oder an der zweiten Stirnseite an tiefster Stelle vorgesehen.

Eine einfache und zuverlässig Entleerung ist möglich, wenn bei der erfindungsgemäßen Vorrichtung das liegende Ofengehäuse auf einem Basisgestell in seiner Neigung gegen die Horizontale, zur Auslaßeinrichtung hin nach unten geneigt, verstellbar angeordnet ist.

Die erfindungsgemäße Vorrichtung eignet sich in besonders vorteilhafter Weise zur zuverlässigen und zeitsparenden chemischen Dekontamination und Geruchsneutralisation sowie zur Sterilisation und Erwärmung bzw. Trocknung von Korkgranulat.

Weitere Einzelheiten, Merkmale und Vorteile ergeben sich aus der nachfolgenden Beschreibung eines in der Zeichnung schematisch dargestellten Ausführungsbeispieles der erfindungsgemäßen Vorrichtung. Es zeigen:
- Fig. 1: schematisch eine Seitenansicht der Vorrichtung, und
- Fig. 2: eine Vorderansicht der Vorrichtung gemäß Fig. 1.

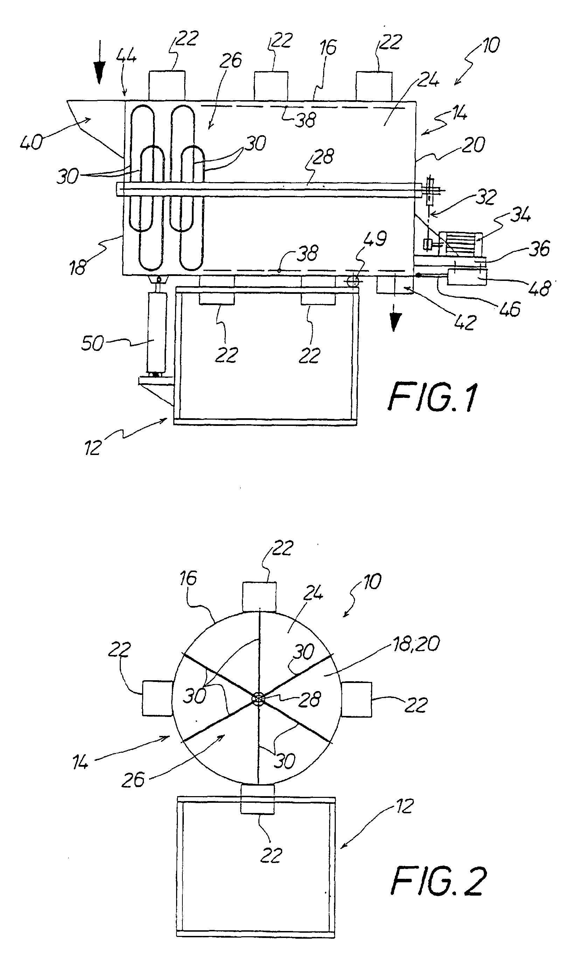

Die Fig. 1 und 2 zeigen in einer schematischen Darstellung eine Ausbildung der Vorrichtung 10 zur Behandlung eines Schüttguts, wie eines Isolierstoffgranulates, eines Kunststoffgranulates, oder dergleichen, insbesondere zur Behandlung von Korkgranulat, das heißt zur Reduktion chemischer Kontaminanten, zur Geruchsneutralisation, zur Sterilisation und zur Erwärmung bzw. Trocknung von Korkgranulat. Die Vorrichtung 10 weist auf einem Basisgestell 12 ein liegendes zylindrisches Ofengehäuse 14 auf. Das Ofengehäuse 14 weist einen zylindrischen Gehäusemantel 16 und eine erste und eine zweite Stirnfläche 18 und 20 auf. Am Umfang des Gehäusemantels 16 sind entlang einer Schraubenlinie voneinander äquidistant beabstandet Mikrowellen-Strahlenquellen 22 vorgesehen.

Im Inneren 24 des zylindrischen, liegenden Ofengehäuses 14 der Vorrichtung 10 ist ein Rührwerk 26 mit von einer hohlen Rührwerkachse 28 radial wegstehenden Rührorganen 30 vorgesehen. Von.den Rührorganen 30 sind nur einige dargestellt. Die Rührwerkachse 28 erstreckt sich zentral zwischen der ersten Stirnfläche 18 und der zweiten Stirnfläche 20 des zylindrischen Ofengehäuses 14 hindurch und ist dort drehbar gelagert. Die Rührwerkachse 28 ist mit Auslaßlöchem für ein gasförmiges Fluid ausgebildet und mittels einer Transmissionseinrichtung 32 mit einem Antriebsmotor 34 wirkverbunden. Der Antriebsmotor 34 ist an einer Konsole 36 angeordnet, die von der zweiten Stirnfläche 20 außenseitig wegsteht.

Die Rührwerkachse 28 ist entlang ihrer gesamten Länge zwischen der ersten und der zweiten Stirnfläche 18 und 20 mit Rührorganen 30 versehen. Das ist durch die beiden strichlierten Linien 38 schematisch angedeutet. Die von der hohlen Rührwerkachse 28 wegstehenden Rührorgane 30 sind als hohle Rührorgane ausgebildet und mit der Rührwerkachse 28 strömungstechnisch verbunden und mit Auslaßlöchem für das gasförmige Fluid versehen. Das gasförmige Fluid wird durch einen Fluid-Einlaßanschluß in die Rührwerkachse 28 eingeleitet.

Das auf dem Basisgestell 12 liegend montierte zylindrische Ofengehäuse 14 weist einen Granulateinlaß 40 und eine Granulatausgabeeinrichtung 42 sowie einen Absauganschluß für das gasförmige Fluid auf. Der Absauganschluß für das gasförmige Fluid kann auch an der hohlen Rührwerkachse 28 vorgesehen sein. Der Granulateinlaß 40 ist am Oberrand 44 der ersten Stirnfläche 18 des liegenden zylindrischen Ofengehäuses 14 vorgesehen. Er ist mit einer (nicht gezeichneten) Verschlußeinrichtung versehen. Die Granulatausgabeeinrichtung 42 ist am Gehäusemantel 16 unterseitig, das heißt an der tiefsten Stelle des Gehäusemantels 16 in der Nachbarschaft der zweiten Stirnfläche 20 des zylindrischen Ofengehäuses 14 vorgesehen. Die Granulatausgabeeinrichtung 42 kann auch an der zweiten Stirnfläche 20 an deren tiefster Stelle, d.h. in der Nachbarschaft der tiefsten Stelle des Gehäusemantels 16 vorgesehen sein. Die Granulatausgabeeinrichtung 42 weist einen Schieber 46 auf, der mit einer Antriebseinrichtung 48 verbunden ist. Die Antriebseinrichtung 48 ist an der Unterseite der Konsole 36 befestigt.

Das liegende zylindrische Ofengehäuse 14 ist um eine zwischen dem Ofengehäuse 14 und dem Basisgestell 12 vorgesehene Lagereinrichtung 49 gegen die Horizontale - zur Auslaßeinrichtung 42 hin nach unten geneigt - verstellbar vorgesehen. Zu diesem Zwecke ist zwischen dem Basisgestell 12 und dem Ofengehäuse 14 ein Antrieb 50 angeordnet, der beispielsweise von einer Kolben-Zylindereinheit gebildet ist.

## Patentansprüche

1. Vorrichtung zur Behandlung eines Schüttguts, insbesondere zur Reduktion chemischer Kontaminanten, zur Sterilisation und zur Trocknung von Korkgranulat durch Bestrahlung mit einer Mikrowellenstrahlung, mit einem liegenden zylindrischen Ofengehäuse (14), in dessen Innerem (24) ein Rührwerk (26) mit von einer Rührwerkachse (28) wegstehenden Rührorganen (30) vorgesehen ist, wobei das Ofengehäuse (14) einen Granulateinlaß (40) und eine Granulatausgabeeinrichtung (42) aufweist, und wobei am Gehäusemantel (16) voneinander beabstandet eine Anzahl Mikrowellen-Strahlenquellen (22) angeordnet sind,
**dadurch gekennzeichnet,**
**daß** die Rührwerkachse (28) als hohle Achse ausgebildet ist, die einen Einlaßanschluß für ein gasförmiges Fluid aufweist, und daß die hohle Rührwerkachse (28) mit Auslaßlöchern für das gasförmige Fluid ausgebildet ist.

2. Vorrichtung nach Anspruch 1,
**dadurch gekennzeichnet,**
**daß** die Mikrowellen-Strahlenquellen (22) am Umfang des Gehäusemantels (16) äquidistant vorgesehen sind.

3. Vorrichtung nach Anspruch 2,
**dadurch gekennzeichnet,**
**daß** die Mikrowellen-Strahlenquellen (22) entlang einer Schraubenlinie angeordnet sind.

4. Vorrichtung nach Anspruch 1,
**dadurch gekennzeichnet,**
**daß** die von der hohlen Rührwerkachse (28) wegstehenden Rührorgane (30) als hohle Rührorgane ausgebildet, mit der Rührwerkachse (28) fluidisch verbunden und mit Auslaßlöchem für das gasförmige Fluid versehen sind.

5. Vorrichtung nach Anspruch 1 - 4,
**dadurch gekennzeichnet,**
**daß** die hohle Rührwerkachse (28) und/oder das Ofengehäuse (14) mit einem Absauganschluß für das gasförmige Fluid versehen ist.

6. Vorrichtung nach Anspruch 1,
**dadurch gekennzeichnet,**
**daß** der Granulateinlaß am Oberrand (44) der einen Stirnseite (18) des liegenden Ofengehäuses (14) und daß die Granulatausgabeeinrichtung (42) am Gehäusemantel (16) unterseitig in der Nachbarschaft der zweiten Stirnseite (20) des liegenden Ofengehäuses (14) oder an der zweiten Stirnseite (20) an deren tiefster Stelle vorgesehen ist.

7. Vorrichtung nach einem der Ansprüche 1 bis 6,
**dadurch gekennzeichnet,**
**daß** das liegende Ofengehäuse (14) auf einem Basisgestell (12) in seiner Neigung gegen die Horizontale, zur Auslaßeinrichtung (42) hin nach unten geneigt, verstellbar angeordnet ist.
